# EUROPEAN PATENT SPECIFICATION

(11) **EP 1 162 990 B1**
(45) Date of publication and mention of the grant of the patent: **05.11.2008**
(21) Application number: 00916561.4
(22) Date of filing: 20.03.2000
(51) Int. Cl.: A61K 38/06, A61K 38/07, A61P 9/10, A61P 11/00, A61P 17/02

(54) **TREATMENT WITH SMALL PEPTIDES TO EFFECT ANTIFIBROTIC ACTIVITY**
BEHANDLUNG MIT KLEINEN PEPTIDEN UM EINE ANTIFIBROSEAKTIVITÄT ZU BEWIRKEN
TRAITEMENT A BASE DE PETITES PEPTIDES INDUISANT UNE ACTIVITE ANTI-FIBROSE

(30) Priority: 22.03.1999 US 125514 P
(43) Date of publication of application: 19.12.2001
(73) Proprietor: Mowycal Lending, LLC, Phoenix, Arizona 85011 (US)
(72) Inventor: CLAGETT, James, Snohomish, WA 98290 (US)
(74) Representative: Hofer, Dorothea
(86) International application number: PCT/US2000/007411
(87) International publication number: WO 2000/056349

(56) References cited:
- ADAMSON I Y ET AL: "Enhanced clearance of silica from mouse lung after instillation of a leukocyte chemotactic factor." EXPERIMENTAL LUNG RESEARCH, (1994 MAY-JUN) 20 (3) 223-33. , XP001041795
- BOTHA ET. AL.: 'Postinjury neutrophil priming adn activation: An early vunerable window' AN EARLY VUNERABLE WINDOW SURGERY vol. 118, August 1995, pages 358 - 365, XP002929377
- LAWRENCE ET. AL.: 'Decreased polymorphonuclear leucocyte chemotatic response to leukotriene B4 in cystic fibrosis' CLINICAL EXPERIMENTAL IMMUNOLOGY vol. 89, April 1992, pages 321 - 324, XP002929378
- SASSEN ET. AL.: 'Development and regression of atherosclerosis in pigs, Effects of n-3 Fatty Acids, Their Incorporation Into Plasma and Aortic Plaque Lipids, and Granulocyte Function' ARTERIOSCLEROSIS AND TROMBOSIS vol. 13, no. 5, May 1993, pages 651 - 660, XP002929379
- TOTANI ET. AL.: 'Polymorphonuclear Leukocytes Enhances Release of Growth Factors by Cultured Andothelial Cells' ATERIOSCLEROSIS AND THROMBOSIS vol. 14, no. 1, January 1994, pages 125 - 132, XP002929380
- STRAGLIOTTO ET. AL.: 'Fungctionally abnormal monocytes in hypercholestrolemia' ARTESCLEROSIS AND THROMBOSIS vol. 13, no. 6, June 1993, pages 944 - 950, XP002929382
- MACGREGOR R. R.: 'In Vivo Neutrrophil Delivery in Men with Alcoholic Cirrhosis is Normal Despite Depressed in vitro Chemotaxis' ALCOHOLISM: CLINICAL AND EXPERIMENTAL RESEARCH vol. 14, no. 2, March 1990 - April 1990, pages 195 - 199, XP002929381
- MARCEAU F.: 'Evidence of Vascular Tone Regulation by Resident of Inflitrating Leukocytes' BIOCHEMICAL PHARMACOLOGY vol. 52, no. 10, 1996, pages 1481 - 1488, XP002929383

## Description

### FIELD OF THE INVENTION

This invention relates to the use of small peptides for treating mammals to effect antifibrotic activity and thereby inhibit, prevent or even reverse fibrolysis in the mammal. More particularly, such treatment of mammals exhibiting fibrous lesions in arterial or airway lumens provides lumen remodeling as demonstrated by reduction in fibrosis.

### BACKGROUND OF THE INVENTION

Endothelial dysfunction that results from the injury leads to compensatory responses that alter the normal homeostatic properties of the endothelium. Thus, the different forms of injury increase the adhesiveness of the endothelium with respect to leukocytes or platelets, as well as its permeability. The injury also induces the endothelium to have procoagulant instead of anticoagulant properties and to form vasoactive molecules, cytokines, and growth factors.

If the inflammatory response does not effectively neutralize or remove the offending agents, it can continue indefinitely. In doing so, the inflammatory response stimulates migration and proliferation of smooth-muscle cells that become intermixed with the area of inflammation to form an intermediate lesion. If these responses continue unabated, they can thicken the artery wall, bronchial airway, or other inflamed lumen.

Typically, in atherosclerosis, the lumen compensates by gradual dilation, so that up to a point, the lumen remains unaltered. As for the inflammatory cells, granulocytes are rarely present during any phase of atherogenesis. Instead, the response is mediated by monocyte-derived macrophages and specific subtypes of T lymphocytes at every stage of the disease.

Continued inflammation results in increased numbers of macrophages and lymphocytes, which both emigrate from the blood and multiply within the lesion. Activation of these cells leads to the release of hydrolytic enzymes, cytokines, chemokines, and growth factors, which can induce further damage and eventually lead to focal necrosis. Thus, cycles of accumulation of mononuclear cells, migration and proliferation of smooth muscle cells, and formation of fibrous tissue lead to further enlargement and restructuring of the lesion, so that it becomes covered by a fibrous cap that overlies a core of lipid and necrotic tissue - a so-called advanced, complicated lesion. At some point, the artery can no longer compensate by dilation. Then, the lesion intrudes into the lumen and alters the flow of blood.

The cellular interactions in atherogenesis are fundamentally no different from those in chronic inflammatory-fibroproliferative diseases such as cirrhosis, rheumatoid arthritis, glomeruloselerosis, pulmonary fibrosis, and chronic pancreatitis. The response of each particular tissue or organ depends on its characteristic cells and architecture, its blood and lymph supply, and the name of the offending agents. Thus, the cellular response in the arteries (atherosclerosis), liver (cirrhosis), joints (rheumatoid arthritis), kidneys (glomcrulosclerosis), lungs (pulmonary fibrosis), and pancreas (pancreatitis) are similar yet are characteristic of each tissue or organ.

Asthma is characterized by a complex inflammatory response of airway eosinophilia, edema, mucus hypersecretion, bronchial epithelial injury and hyperreactivity. Inhaled allergen challenge in allergic asthmatics provokes an immediate airway hypersensitivity reaction, an early airway response (EAR), that is frequently followed several hours later by a delayed airway reaction, a late phase airway response (LAR).

Insights into the mechanisms of chronic inflammation in asthma have come from the investigation of the LAR in animal models. A number of animal models have been shown to produce typical features of LAR in a number of species including mouse, rat, guinea pig and non-human primates.

Balb/C mice have been reported to produce allergic pulmonary disease mimicking human allergic pulmonary disease including airspace/interstitial eosinophilia, mucous secretion, edema and airway constriction in response to immunization with ovalbumin (OVA) after repeated challenge. The structure and function of pulmonary tissue changes in chronic asthma have been reported in human lung biopsies and autopsy materials. Persistent inflammation, deposition of fibrotic collagen in the interstitium, and airway narrowing characterize these structural changes. Reduced pulmonary efficiency is a hallmark of the functional changes associated with chronic asthma. It has been shown that immunization of Balb/ C mice with OVA for three months or more produces a similar pathology to that of human chronic asthma with fibrosis, airway narrowing and persistent inflammatory infiltration.

Granulocytes are rare in atherosclerosis, and among cirrhosis, glomeruloselerosis, and chronic pancreatitis. They are present only in rheumatoid arthritis and pulmonary fibrosis. In the case of arthritis, although the early response begins with granulocytes, they are found primarily within the joint cavity. Macrophages and lymphocytes predominate in the synovium, leading to erosion of cartilage and bone, which is replaced by fibrosis tissue (pannus). In pulmonary fibrosis, granulocytes initially appear in the alvcolar spaces; however, the lung parenchyma, where fibrosis ultimately occurs, is infiltrated by macrophages and lymphocytes. Thus, there are parallels among these inflammatory diseases. At least three different types of macrophages, each regulated by different T-cell cytokines (interferon-7, interleukin-2, interleukin-4, and interleukin-10) have been identified.

Chronic inflammatory responses are often associated with specific types of injurious or granuloma-inducing agents. If the injurious agent or agents are not removed or nullified by the inflammatory response and the inflammation progress, the response changes from a protective to an injurious response. Such constant or repetitive injury can stimulate each tissue to repair or wall off the damage by means of a fibropro-liferative response, which, when excessive, diminishes the functional capacity of the tissue or organ and becomes part of the disease process.

Fibrosis is a conspicuous feature of chronically inflamed tissue. It is characterized by progressive and excessive accumulation of extracellular matrix collagen as a consequence of increased proliferation of fibroblasts (the major mesenchymal cell responsible for the synthesis of interstitial collagen). A feature of lung tissue from patients with fibrotic lung disease is an increased number of mast cells, many of which are in a state of partial degranulation located in close proximity to proliferating fibroblasts.

Collagen types I, II, III, V, and XI, representing the major interstitial collagen's, are composed of one to three α-chains, each of 95-100kD molecular weight, associated to form a single triple helix structure. Type I collagen has an asymmetric heterotrimeric configuration composed of α1 and α2 chains in a stoichiometric proportion of 2:1, giving rise to the chain composition (α₁[I]₂, α₂[I]) while type III collagen, a homotrimer of three α1 chains has the designation [α₁(I)]₃. The α1 and α2 chains of type I collagen migrate at slightly different rates under reducing conditions. The collagen stimulated by tryptase treatment was identified as type I on the basis of the α chain composition, and by immunoblotting with a specific antibody to type I collagen.

The collagens form an integral part of the extracellular matrix, and the amount of collagen deposited in the lung is tightly regulated to ensure a strict balance between biosynthesis and degradation. An inappropriate control of this balance can lead to enhanced collagen deposition resulting in fibrosis. It is now recognized that any alterations in either the amounts of collagen as reported in acute respiratory distress syndrome, cryptogenic fibrosing alveolitis, sarcoidosis or, indeed, the type of collagens, may contribute to cellular abnormalities in the lung.

The normal human lung is composed of 65% type I and 30% type III collagen. A specific increase in the amount of type I collagen, with a concomitant decrease in type III, has been reported in idiopathic chronic pulmonary fibrosis, and in fibrosis associated with atherosclerosis, and liver cirrhosis. In general, compliant tissues have a low ratio of type I to type III collagen. Less compliant tissues, such as that found in pulmonary fibrosis, have a higher ratio.

Smooth-muscle cells in the media of arteries, as well as in lesions, are surrounded by different types of connective tissue. In the media of arteries, the matrix consists largely of type I and III fibular collagen, whereas in the lesions of atheroselerosis it consists largely of proteoglyean, intermixed with loosely scattered collagen fibrils.

I.Y.R. Adamson et al. in Experimental Lung Research to: 223-233 (1994) describe an enhanced clearance of silica from mouse Lung after administration of the small peptide N-formyl-L-methionyl-leucyl-phenylalanine (FMLP). The subsequent reduction in particle content of the lung is associated with a lower level of pulmonary fibrosis.

New concepts for the treatment of fibrosis that results from inflammation caused by various disease states, trauma or surgical procedures are continually being sought.

### SUMMARY OF THE INVENTION

It has been discovered that fibrosis in mamals can be treated by the administration to such mammals of pharmaceutical compositions containing in a suitable pharmacological carrier a small peptide having antifibrotic activity. Such treatment can inhibit fibrosis. In preferred embodiments of the present invention, fibrosis is reduced or reversed, thereby providing lumens having little constriction of the passageway and fluid flow therein.

Accordingly, the present invention provides the use as defined in claim 1. Preferred embodiments are defined in the sub-claims.

In the present invention, a pharmaceutical compositions containing in a suitable pharmacological carrier an antifibrotic effective amount of aN-formyl- methionyl-leucyl("f-Met-Leu") peptide having antifibrotic activity can be used, namely those having the formula f-Met-Leu-X where X is selected from the group consisting of Tyr, Tyr-Phe, Phe-Phe and Phe-Tyr.

The present invention is used for treatment of mammals for a variety of fibrotic disease conditions selected from the group consisting of pulmonary fibrosis caused by chronic asthma, atherosclerosis, cirrhosis, glomerulosclerosis, chronic pancreatitus and coronary artery disease (such as caused by infection by bacterium Chlamydia pneumoniae). Excessive fibrosis due to trauma or to surgical procedures can also be treated by methods of the present invention including, for example, post-operative fibrosis peri-neurally in the dura or nerve roots following spinal surgery, tenolysis of injured or repaired tendons with adhesions, neurolysis of damaged or repaired peripheral nerves with adhesions, post-operative adhesions from gynecologic and abdominal surgeries, reparative surgery of the vas deferens or fallopian tubes for reversal of male or female sterilization, and surgical repair of other tubular structures such as urethra, intestine or esophagus.

For treating airway membranes, a preferred mode of administration is by inhalation. For treating surface lesions, a preferred mode of administration is topical application using a suitable pharmacological carrier. Intradermal injection or tablets can be used for systemic treatments.

In certain preferred embodiments of the present invention, patients can benefit by administering the peptide of the present invention in combination with a second active ingredient. Particularly useful other active ingredients for such combination in accord with the present invention are, for example, antileukotrienes, beta2 agonists, corticosteroids, and the like.

### BRIEF DESCRIPTION OF DRAWINGS

FIG. 1 is schedule illustrating immunization to induce chronic asthma and subsequent treatment in accord with the present invention.
FIG. 2A and 2B are photographs illustrating a comparison of pathological features of lung tissue of mice with chronic asthma after treatment (A) in accord with the present invention, and without treatment (B).
FIG. 3A-3C are photographs illustrating a comparison of lung tissue of mice exhibiting a fibrotic accumulation of collagen after treatment (A) in accord with the present invention, without treatment (C), and control (B).
FIG. 4A-4C are photographs illustrating a comparison of lung tissue of mice exhibiting a fibrotic accumulation of collagen after treatment (B) in accord with the present invention, without treatment (C), and control (A).
FIG. 5 is a graph illustrating the effects of treatment in accord with the present invention on airway mucous plug and inflammatory cell accumulation in mice induced with chronic asthma.
FIG. 6 is a graph illustrating the effects of treatment in accord with the present invention on lung mucous cell number in airways of mice induced with chronic asthma.
FIG. 7 is a graph of the effects of treatment in accord with the present invention on eosinophil and neutrophil numbers in airways of mice induced with chronic asthma.
FIG. 8 is a graph of the effects of treatment in accord with the present invention on granuloma in lungs of mice induced with chronic asthma.
FIG. 9A-9C are photographs illustrating no hepatic toxicity is induced by treatment in accord with the present invention of mice induced with chronic asthma; after treatment (A) in accord with the present invention, without treatment (C), and control (B).
FIG. 10 is schedule illustrating immunization to induce chronic asthma and subsequent treatment in accord with the present invention prior to additional intranasal challenges.
FIG. 11A-11C are photographs illustrating a comparison of lung tissue of mice with chronic asthma and subsequently further challenged intranasally, after treatment (A) in accord with the present invention, without treatment (C), and control (B).
FIG. 12A-12C are photographs illustrating a comparison of collagen in lung tissue of mice with chronic asthma and subsequently further challenged intranasally, after treatment (A) in accord with the present invention, without treatment (C), and control (B).
FIG. 13A-13C are photographs illustrating a comparison of mucous cells in lung tissue of mice with chronic asthma and subsequently further challenged intranasally, after treatment (A) in accord with the present invention, without treatment (C), and control (B).
FIG. 14 a graph of the effects of treatment in accord with the present invention on eosinophil and neutrophil numbers in airways of mice induced with chronic asthma and subjected to repeated allergic challenge.
FIG. 15 is a graph illustrating the effects of treatment in accord with the present invention on airway mucous plug and inflammatory cell accumulation in mice induced with chronic asthma and subjected to repeated allergic challenge.
FIG. 16 is a graph illustrating the effects of treatment in accord with the present invention on mucous plug formation in airways of mice induced with chronic asthma and subjected to repeated allergic challenge.
FIG. 17 is a graph illustrating the effects of treatment in accord with the present invention on lung mucous cell number in airways of mice induced with chronic asthma and subjected to repeated allergic challenge.
FIG. 18 is a graph of the effects of treatment in accord with the present invention on granuloma in lungs of mice induced with chronic asthma and subjected to repeated allergic challenge.

### DETAILED DESCRIPTION OF THE INVENTION

In accord with the present invention, certain small peptides having the formulaf-Met-Leu-X where X is selected from the group consisting of Tyr, Tyr-Phe, Phe-Phe and Phe-Tyr have been found to have surprising activity for inhibiting fibrosis and, in preferred embodiments reducing fibrous lesions and remodeling lumens. In connection with this invention the term "remodeling" is used to mean that the lumen, e. g., an airway passage or artery, or the like, is restructured back toward its original condition before the pathological effect. In highly preferred embodiments, the lumen is returned to its original condition prior to disease. As a result, such peptides are used for treatment of mammals exhibiting fibrous lesions selected from the group consisting of pulmonary fibrosis caused by chronic asthma, atherosclerosis, cirrhosis, glomerulosclerosis, chronic pancreatitus and coronary artery disease. Excessive fibrosis due to trauma or to surgical procedures can also be treated by methods of the present invention including, for example, post-operative fibrosis perineurally in the dura or nerve roots following spinal surgery, tenolysis of injured or repaired tendons with adhesions, neurolysis of damaged or repaired peripheral nerves with adhesions, post-operative adhesions from gynecologic and abdominal surgeries, reparative surgery of the vas deferens or fallopian tubes for reversal of male or female sterilization, and surgical repair of other tubular structures such as urethra, intestine or esophagus.

The peptides to be used in this invention can be prepared by conventional small peptide chemistry techniques. The peptides when used for administration are prepared under aseptic conditions with a pharmaceutically acceptable carrier or diluent.

Doses of the pharmaceutical compositions will vary depending upon the subject and upon the particular route of administration used. Dosages can range from 0.1 to 100,000 µg/kg a day, more preferably 1 to 10,000 µg/kg.

Most preferred dosages range from about 1 to 100 µg/kg of body weight, more preferably from about 1 to 10 µg/kg and most preferably 1.0 to 2.0 µg/kg. Doses are typically administered from once a day to every 4-6 hours depending on the severity of the condition. For acute conditions, it is preferred to administer the peptide every 4-6 hours. For maintenance or therapeutic use, it may be preferred to administer only once or twice a day. Preferably, from about 0.18 to about 16 mg of peptide are administered per day, depending upon the route of administration and the severity of the condition. Desired time intervals for delivery of multiple doses of a particular composition can be determined by one of ordinary skill in the art employing no more than routine experimentation.

Routes of administration include oral, parenteral, rectal, intravaginal, topical, nasal, ophthalmic, direct injection, etc. In a preferred embodiment, the peptides of this invention are administered to the patient in an anti-inflammatory effective amount or in a dosage that inhibits degranulation of mast cells. An exemplary pharmaceutical composition is a therapeutically effective amount of a peptide in accord with the present invention that provides anti-inflammatory effect or that inhibits degranulation of mast cells, typically included in a pharmaceutically acceptable carrier.

The term "pharmaceutically acceptable carrier" as used herein, and described more fully below, includes one or more compatible solid or liquid filler diluents or encapsulating substances that are suitable for administration to a human or other animal. In the present invention, the term "carrier" thus denotes an organic or inorganic ingredient, natural or synthetic, with which the molecules of the invention are combined to facilitate application. The term "therapeutically-effective amount" is that amount of the present pharmaceutical compositions, which produces a desired result or exerts a desired influence on the particular condition being treated. Various concentrations may be used in preparing compositions incorporating the same ingredient to provide for variations in the age of the patient to be treated, the severity of the condition, the duration of the treatment and the mode of administration.

The carrier must also be compatible. The term "compatible", as used herein, means that the components of the pharmaceutical compositions are capable of being commingled with a small peptides of the present invention, and with each other, in a manner such that does not substantially impair the desired pharmaceutical efficacy.

The small peptides to be used according to the invention are typically administered per se (neat). However, they may be administered in the form of a pharmaceutically acceptable salt. Such pharmaceutically acceptable salts include, but are not limited to, those prepared from the following acids: hydrochloric, hydrobromic, sulfuric, nitric, phosphoric, maleic, acetic, salicylic, p-toluene-sulfonic, tartaric, citric, methanesulphonic, formic, malonic, succinic, naphthalene-2-sulfonic, and benzenesulphonic. Also, pharmaceutically acceptable salts can be prepared as alkaline metal or alkaline earth salts, such as sodium, potassium or calcium salts of the carboxylic acid group. Thus, the present invention provides pharmaceutical compositions, for medical use, which comprise peptides of the invention together with one or more pharmaceutically acceptable carriers thereof and optionally any other therapeutic ingredients.

The compositions include those suitable for oral, rectal, intravaginal, topical, nasal, ophthalmic or parenteral administration, all of which may be used as routes of administration using the materials of the present invention. Pharmaceutical compositions containing peptides of the present invention may also contain one or more pharmaceutically acceptable carriers, which may include excipients such as stabilizers (to promote long term storage), emulsifiers, binding agents, thickening agents, salts, preservatives, solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the peptide of this invention, its use in pharmaceutical preparations is contemplated herein. Supplementary active ingredients can also be incorporated into the compositions used for the present invention.

Compositions suitable for oral administration are preferred for treatment of asthma. Typically, such compositions are prepared as an inhalation aerosol, nebule, syrup or tablet. Compositions suitable for topical administration are preferred for treatment of arthritis, although oral compositions also can be convenient. Typically, such topical compositions are prepared as a cream, an ointment, or a solution. The concentrations of the peptide active ingredient in such compositions is typically less than 50 µg/ml, more preferable less than 30 µg/ml, and most preferably from about 5 to 10 µg/ml.

The compositions may conveniently be presented in unit dosage form and may be prepared by any of the methods well known in the art of pharmacy. Methods typically include the step of bringing the active ingredients of the invention into association with a carrier that constitutes one or more accessory ingredients.

Compositions to be used according to the present invention suitable for inhalation administration may be presented, for example, as aerosols or inhalation solutions. An example of a typical aerosol composition consists of the desired quantity of microcrystalline peptide suspended in a mixture of trichloromonofluoromethane and dichlorodifluoromethane plus oleic acid. An example of a typical solution consists of the desired quantity of peptide dissolved or suspended in sterile saline (optionally about 5 % v/v dimethylsulfoxide ("DMSO") for solubility), benzalkonium chloride, and sulfuric acid (to adjust pH).

Compositions to be used according to the present invention suitable for oral administration also may be presented as discrete units such as capsules, cachets, tablets or lozenges, each containing a predetermined amount of the peptide of the invention, or which may be contained in liposomes or as a suspension in an aqueous liquor or non-aqueous liquid such as a syrup, an elixir, or an emulsion. An example of a tablet formulation base includes corn starch, lactose and magnesium stearate as inactive ingredients. An example of a syrup formulation base includes citric acid, coloring dye, flavoring agent, hydroxypropylmethylcellulose, saccharin, sodium benzoate, sodium citrate and purified water.

Compositions suitable for parenteral administration conveniently comprise a sterile aqueous preparation of the molecule to be used for the invention, which is preferably isotonic with the blood of the recipient. This aqueous preparation may be formulated according to known methods using those suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In aqueous solutions, up to about 10 % v/v DMSO or Trappsol can be used to maintain solubility of some peptides. Also, sterile, fixed oils may be conventionally employed as a solvent or suspending medium. For this purpose, a number of fixed oils can be employed including synthetic mono- or diglycerides. In addition, fatty acids (such as oleic acid or neutral fatty acids) can be used in the preparation of injectibles. Further, Pluronic block copolymers can be formulated with lipids at 4° C for compound injection on a time release basis from solid form at 37° C over a period of weeks or months.

Compositions suitable for topical administration may be presented as a solution of the peptide in Trappsol or DMSO, or in a cream, ointment, or lotion. Typically, about 0.1 to about 2.5 % active ingredient is incorporated into the base or carrier. An example of a cream formulation base includes purified water, petrolatum, benzyl alcohol, stearyl alcohol, propylene glycol, isopropyl myristate, polyoxyl 40 stearate, carbomer 934, sodium lauryl sulfate, acetate disodium, sodium hydroxide, and optionally DMSO. An example of an ointment formulation base includes white petrolatum and optionally mineral oil, sorbitan sesquioleate, and DMSO. An example of a lotion formulation base includes carbomer 940, propylene glycol, polysorbate 40, propylene glycol stearate, cholesterol and related sterols, isopropyl myristate, sorbitan palmitate, acetyl alcohol, triethanolamine, ascorbic acid, simethicone, and purified water.

### The OVA-induced Bronchial Asthma Mouse Model

The chronic murine model of asthma was used to test the effects of f-Met-Leu-Phe-Phe (HK-X) on persistent asthma. This murine system is a model of chronic asthma where there is enormous infiltration of inflammatory cells in association with outer boundaries of the airway basal lamina. An immunization period of five (5) months during which mice were subjected to an initial immunization procedure during the first month followed by weekly intranasal challenges with OVA established a condition of persistent asthma in the mice. Immunized mice contained increased accumulations of collagen around blood vessels and airways, demonstrating a fibrotic condition. Immunized mice were treated with HK-X eight times over a sixteen day period (i.e., every other day).

### EXAMPLE 1

### Materials and Methods

Reagents: Crystalline OVA was obtained from Pierce Chem. Co. (Rockford, IL) and aluminum potassium sulfate (alum) from Sigma Chem. Co. (St. Louis, MO), and pyrogen-free distilled water from Baxter, Healthcare Corporation (Deerfield, IL). The OVA (500 µg/ml) was mixed with equal volumes of 10% (wt/vol) alum in distilled water. The mixture (adjusted to pH 6.5 using 10 N NaOH) after incubation for 60 minutes at room temperature underwent centrifugation at 750 g for 5 minutes; the pellet was resuspended to the original volume in distilled water and used within one hour.

Female BALB/c mice (6-8 wk of age at purchase; D and K, Seattle WA) were housed under conventional conditions for the studies.

### Allergen Immunization/ Challenge Protocols For Inducing Chronic Asthma:

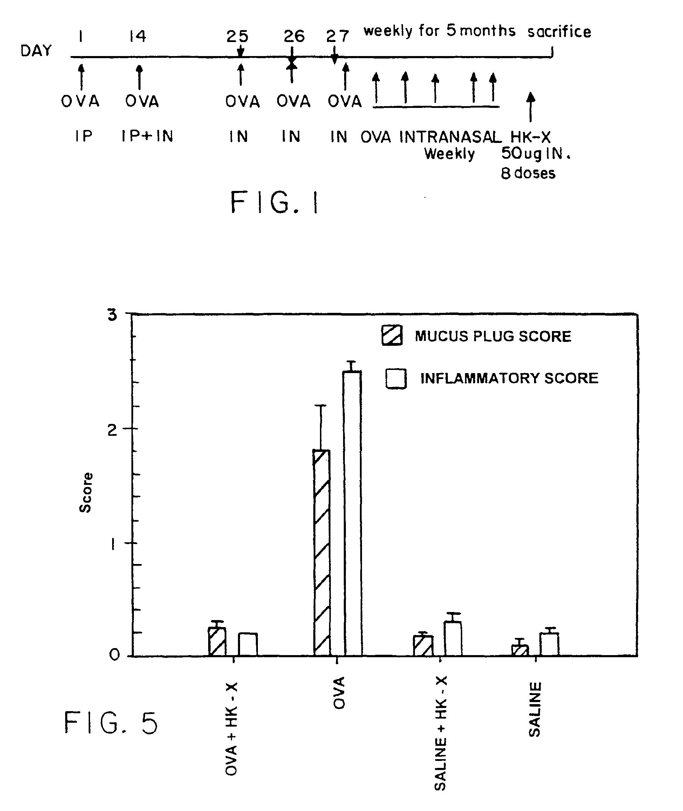

Mice received an intraperitoneal ("i.p.") injection of 0.2 ml (100µg) of OVA in alum on day 1 and i.p. injection (100µg of OVA in alum) combined with intranasal OVA (100µg in saline) on day 14. On days 25, 26 and 27, the mice were challenged further with intranasal OVA (100µg in saline). Subsequently, the mice were challenged weekly with intranasal OVA (100µg in saline) for an additional five months. See FIG. 1.

### Treatment of Chronic Asthma

As indicated in FIG. 1, 50 µg of HK-X was administered intranasally ("IN") for a total of 8 dosages delivered over a period of 16 days. The animals were sacrificed 1 day after the last HK-X dose.

### Preparation of HK-X for Administration

50 µg of HK-X (in 50 µl of saline containing less than 2.5% DMSO). Sufficient DMSO is used to dissolve the compound, but not more than 2.5% by volume. The solution was infused into the lung through the nose while the recipient was anesthetized.

For control vehicle, animals received the same amount of saline through the nose (intranasal) while anesthetized.

### Histology

All animals were sacrificed after the last HK-X treatment or last OVA challenge by the injection of anesthetic. Lungs were excised and fixed with 10% formalin. One lung lobe was fixed in Carnoy's fixative for immunocytochemistry studies. After 24-hr fixation, lungs were dehydrated and put into paraffin blocks. All the paraffin blocks were coded with laboratory numbers for blind analysis.

Paraffin blocks were sectioned and 2 levels of each lung separated by 1-mm distance were selected and 8 slides were made of each level. Slides were stained with Hematoxylin and eosin to visualize general cellular and tissue organization, Alcian blue for the identification of mucus containing cells and mucus released into airways, Mason's trichrome stain for collagen deposition in fibrosis, and Methylene blue and eosin to identify eosinophils in lung tissue.

*Morphometric Analysis*: The following parameters of allergic chronic pulmonary disease were analyzed.
1. Airway Plug scores - by using a scoring system from + to ++++, the severity of mucus secretion into medium and large-sized airways could be measured according to the previous report (Henderson et al., J. Exp. Medicine, vol. 1-84, pp. 1483-94, Oct. 1996).
2. Fraction of Epithelial Cells Containing Mucous Granules - was evaluated by randomly counting the number of epithelial cells containing mucus out of 100 epithelial cells in medium to large airways (600 µm to 1,000 µm diameters). A total of 10 fields were counted in different lung lobes and the mean score was tabulated.
3. Cell Density of Infiltrating Cells - the accumulated inflammatory cells (neutrophils, eosinophils, monocytes and lymphocytes) in association with airways and located in the perivascular compartment was evaluated by using a scoring system ranging from + to ++++. A score of + indicates an inflammatory cell layer of 3 but less than 5 cells; ++ indicates an inflammatory density of 5 cells to 10 cells; +++ indicates an inflammatory density of 10 to 20 cells; and ++++ indicates an inflammatory density of 20 to 40 cells.
4. Numbers of various cell types - eosinophils and neutrophils were quantified by counting the numbers per high power field (40x) in an area of 2,200 u² in association with airways.
5. Granuloma Score - granuloma-like structures were counted at low power (5x). Cellular aggregation associated with either airways or blood vessels was counted as granuloma.

*Statistical Analyses of Histomorphometric Data*: SigmaStat version 2.0 was used to perform statistical comparisons between experimental and control groups. Differences were analyzed for significance (p<0.05) by ANOVA. SigmaPlot version 4.0 or GraphPad Prism was employed for the construction of graphical representation of the data.

### Results:

No adverse reactions or signs of sickness were observed during the immunization of mice with OVA and the subsequent treatment with HK-X. The mice were active during the entire experimental period.

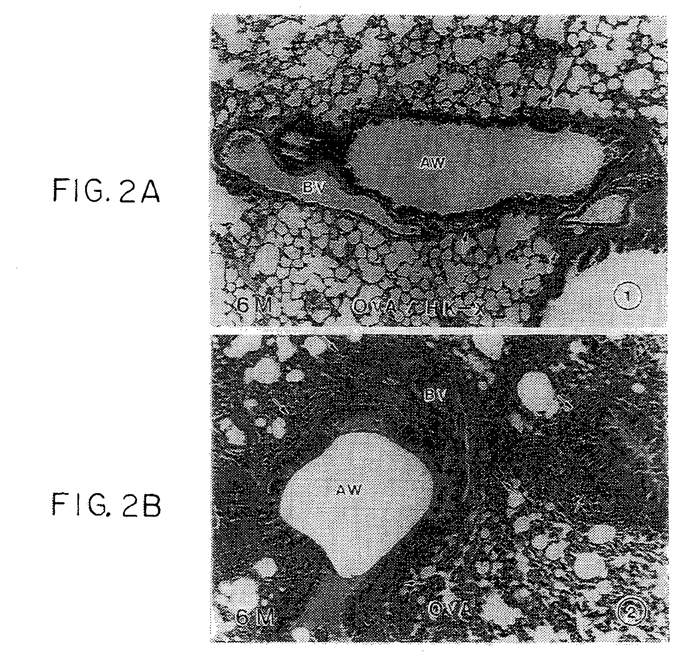

However, after the animals were sacrificed and histological examination performed on the lung tissues, animals immunized with OVA only contained severe pulmonary pathological changes consistent with chronic asthma observed in humans. Thus, this murine system is a model of chronic asthma where there was an enormous infiltration of inflammatory cells in association with outer boundaries of the airway basal lamina (see Fig. 2B).

When animals were treated with 8 doses of HK-X over a 16-day period, the number of inflammatory cells were clearly reduced around airways and blood vessels (see Fig.2A).

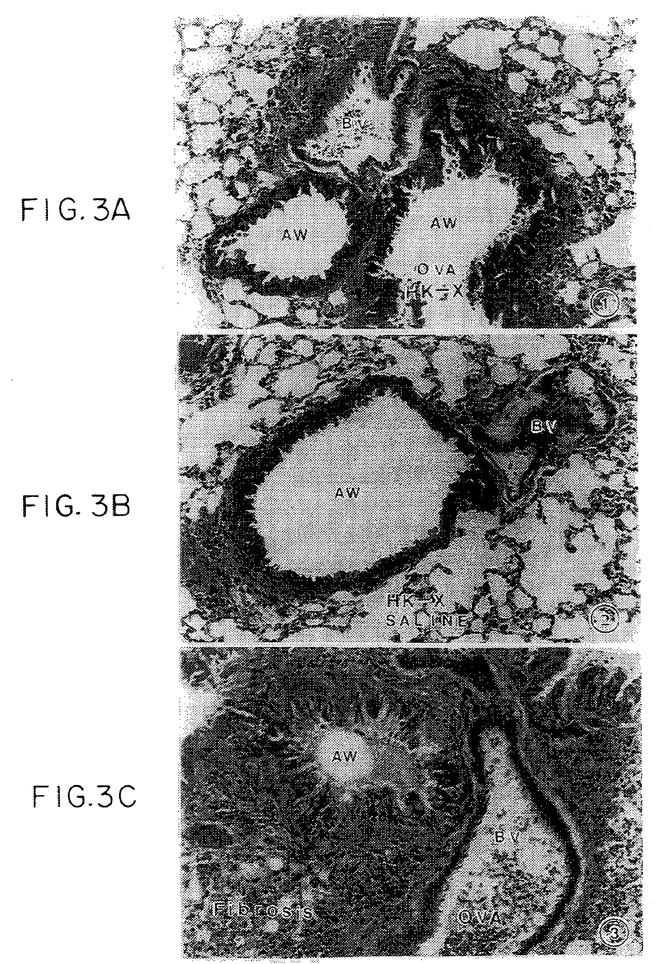

As illustrated in Fig. 3, OVA immunized mice contained increased accumulations of collagen (blue color) around vessels and airways (see Fig. 3C). However, lungs treated with HK-X demonstrated a reduced level of collagen deposition (see Fig. 3A). In control mice (administered HK-X in saline), the pulmonary tissue was free of inflammatory cells and fibrotic collagen deposits (see Fig. 3B).

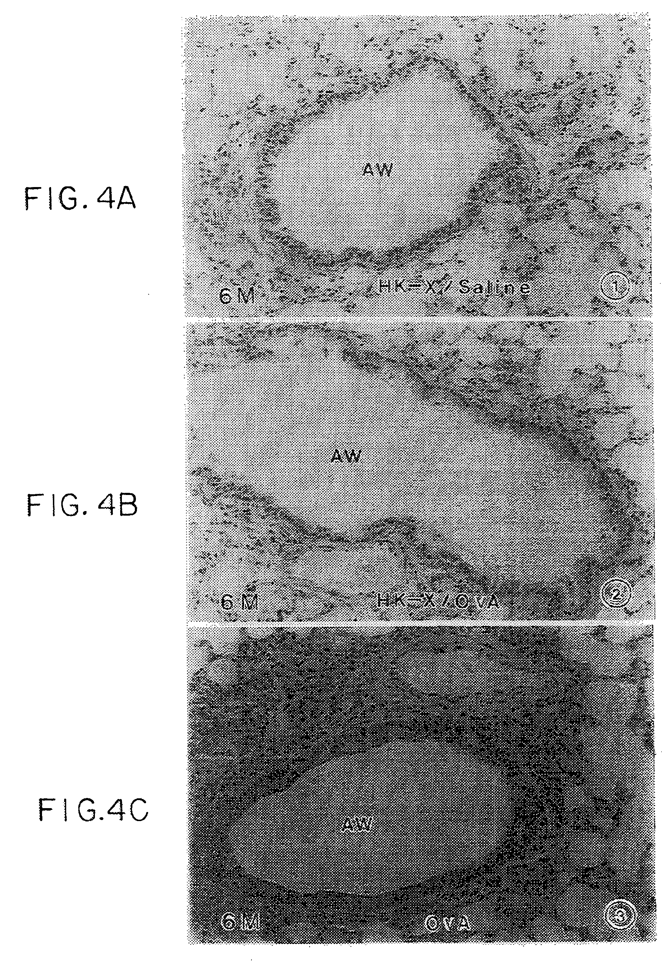

A similar pattern of results was observed when Alcian blue at pH 2.3 visualized mucus-containing cells. A very high proportion of epithelial cells in OVA immunized mice contained mucus granules (see Fig. 4C). By contrast, treatment with HK-X dramatically reduced the numbers of mucus containing cells in the airways (see Fig. 4B). In fact, the frequency was not different from that of control or non-immunized animals given only saline containing HK-X (see Fig. 4A).

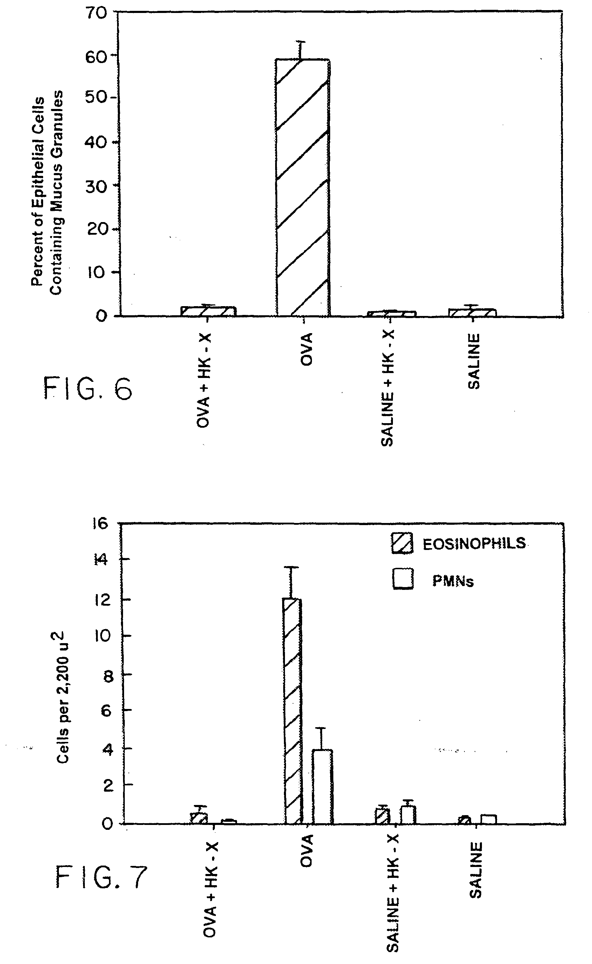

Fifty-to-sixty percent of the airways of these 6 month-old chronic asthmatic mice were plugged with mucus (Fig. 5). When given 8 doses of HK-X intranasally over a 16-day period, there was a remarkagle reduction in mucus accumulation and mucus cells in the airway (Fig. 6). Also, the numbers of infiltrating inflammatory cells - including eosinophils and neutrophils - per unit area was also reduced (Fig. 7). The histopathological observations of OVA immunized and HK-X treated animals resembled those of animals treated with saline or saline containing HK-X only.

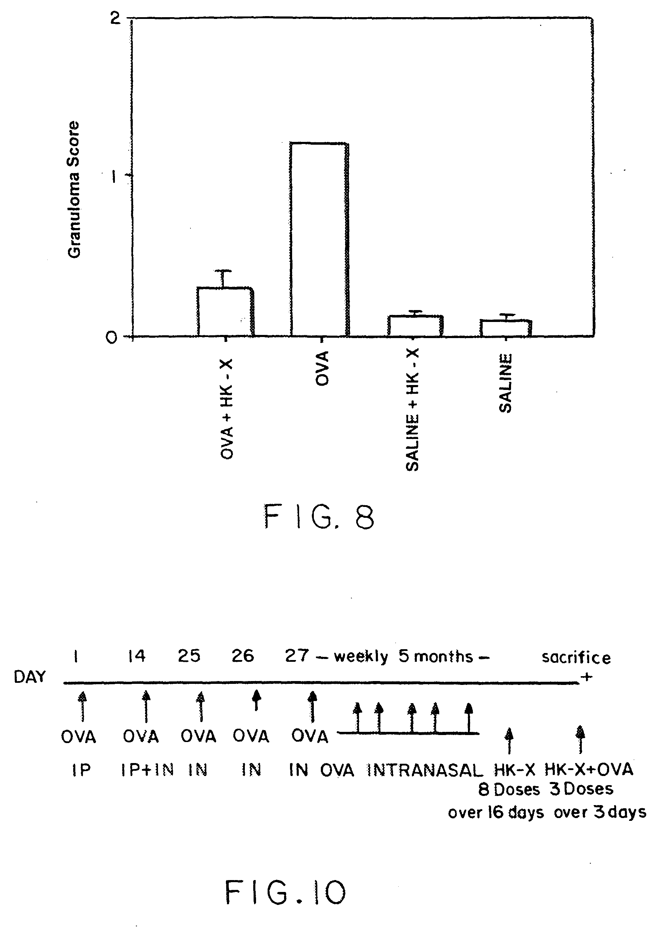

One of the important characteristics of chronic asthma in the murine model is the appearance of granulomatous structures in the lung. HK-X reduces the numbers and sizes of these structures in the lungs of treated animals (Fig. 8).

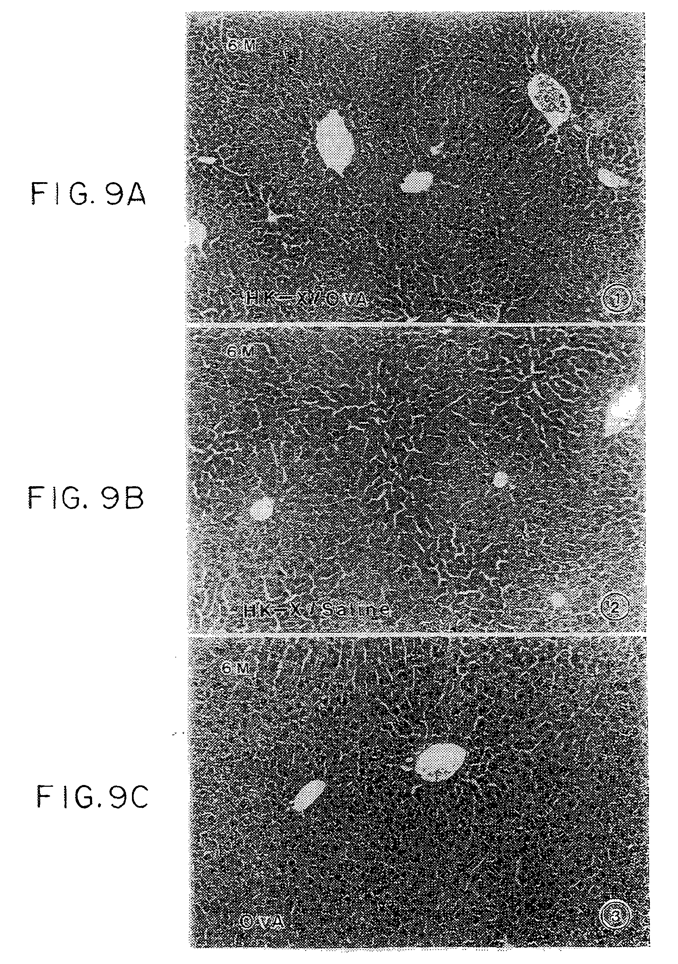

In comparison, for both chronic asthmatic mice treated with HK-X as well as animals treated with normal saline, there are not obvious differences in the histopathology of livers (Fig. 9). Therefore, as determined here, there is no hepatic toxicity produced by frequent intranasally administered doses of HK-X in mice.

These studies were designed to determine whether or not the administration of HK-X reduced airway inflammation and hyperactivity of mucus cells in a murine model of allergen-induced chronic asthma. In this model, mice were sensitized to OVA and exposed to OVA via intranasal route weekly for 5 months and were treated with HK-X intranasally 8 times over a 16 day period. This allergen immunization and challenge regimen lead to a chronic airway infiltration of eosinophilis and other types of inflammatory cells, accumulation of mucus in the airways and hyperplasia of mucus secreting cells.

The airway hypersecretion, hyperplasia of mucus cells, and recruitment of eosinophils and neutrophils was reduced by the administration of HK-X. These results indicate that HK-X is playing an important role in both the reduction of airway hypersecretion of mucus and the late-phase inflammation that occurs in this allergen-induced model of chronic asthma. Moderation of lung inflammation and reduction of mucus secretion and mucus cell differentiation by HK-X was observed.

Of further interest, HK-X was continuously effective over a 16 day period of treatment which indicates that tachyphylaxis did not occur in this model. Further, the intranasal administration of HK-X did not produce any detectable heptatoxicity when administered to mice over a prolonged period at 1 mg per Kg body weight.

### EXAMPLE 2

In the murine chronic asthma model, the pathological changes closely mimic human disease. Example 1 above illustrated that treatment in accord with the present invention reduced pathological changes caused by chronic asthma, including fibrosis. A remarkable reduction of fibrosis, of the numbers of inflammatory cells about airways, and of mucous cells and mucous release within airways was observed.

This example reports the results of tests wherein mice having induced chronic asthma and having been treated in accord with the present invention are subsequently further challenged intranasally ("IN") with OVA. Materials were the same as in Example 1.

### Methods:

Balb/C mice were repeatedly immunized with OVA over a six month period as in Example 1. Thereafter, the mice were treated with 50 µg of HK-X per dose intranasally for eight doses over a 16 day period, as in example 1. Then, a further regimen administered a 50 µg dose of HK-X 15-30 minutes prior to intranasal OVA challenge. This regimen was repeated for a total of three days. On the day after the last treatment the mice were sacrificed. See Fig. 10.

HK-X was prepared in the same manner as in Example 1.

### Administration of HK-X:

The HK-X/OVA group of animals received HK-X dosage of 0.4 mg/Kg body weight (50 ug per 20 gm body weight per day) while the saline group received the same dosage of HK-X without OVA. Previous pharmokinetic studies indicated that the plasma T½ (half life) of HK-X in the mouse was less than 30 min. Therefore, to maintain the plasma levels during the course of the allergen challenge, these mice received additional HK-X doses intranasally immediately before OVA challenge.

### Lung Histology:

The trachea and left lung (upper and lower lobes) were collected and fixed in 10% formalin at 20°C for 15 hr. After embedding in paraffin, the tissues were cut into 5 µm sections. Eosinophils were stained in the lung tissue with Modified Discombe's Solution. The number of eosinophils per unit airway area (2,000 µm²) was determined by morphometric analysis as previously described (Henderson et al., J. Exp. Medicine, vol. 1-84, pp. 1483-94, Oct. 1996, Su et al., American Review Repetitive Diseases, Vol. 147, pp. 448-56, 1993). Airway mucus and mucus cells were identified by the following staining methods: Methylene blue, Mucicarmine, Toluidine blue, and Alcian blue.

Occlusion of the airway diameter by mucus was assessed on a semiquantitative scale ranging from 0 to 5+. For each mouse, individuals blinded to the protocol design assessed 10 airway sections randomly distributed throughout the left lung for mucus occlusion by morphometric analysis. Each airway section was assigned a score for airway diameter occlusion by mucus based on the following criteria: 0, no mucus; 1+, -10% occlusion; 2+, -30% occlusion; 3+, -50% occlusion; 4+, -80% occlusion; and 5+, -90-100% occlusion.

### Results:

### Light Micrographs of Lung Tissues from Allergically Challenged Mice:

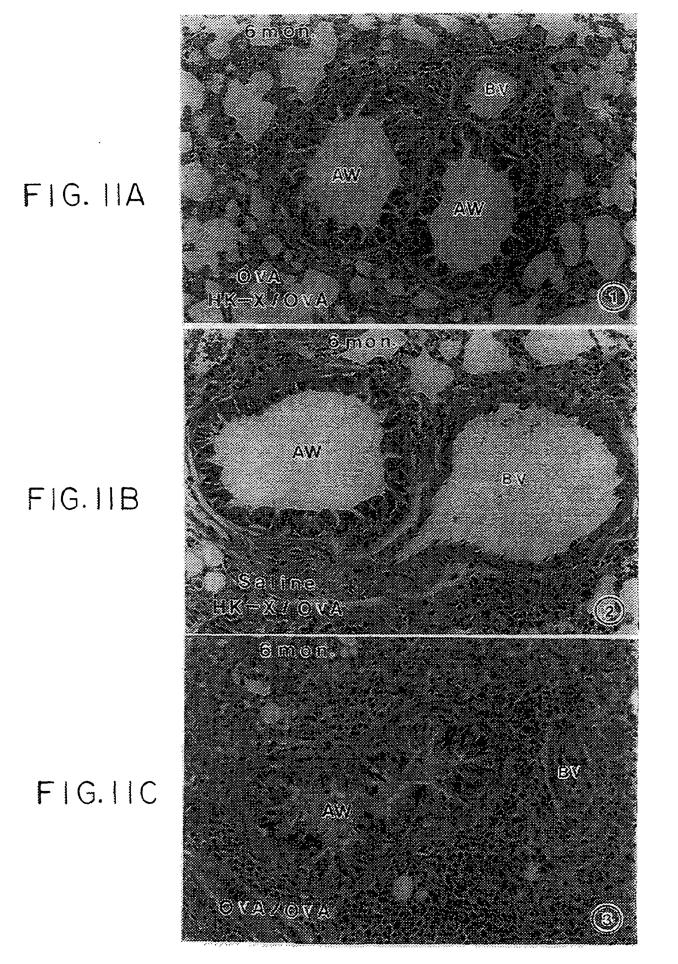

Animals that were immunized with OVA over a 6-month period and, then, repeatedly challenged with OVA contained severe pulmonary structural changes consistent with chronic asthma observed in humans. An enormous infiltration of inflammatory cells was observed in association with outer boundaries of the airway basal lamina (see Fig. 11C). When animals were treated with 8 doses of HK-X over 16 day period and given HK-X 30 min prior to allergic challenge, the number of inflammatory cells were clearly reduced around airways and blood vessels (see Fig. 11A) and was similar to the pattern observed in saline treated mice (see Fig. 11B).

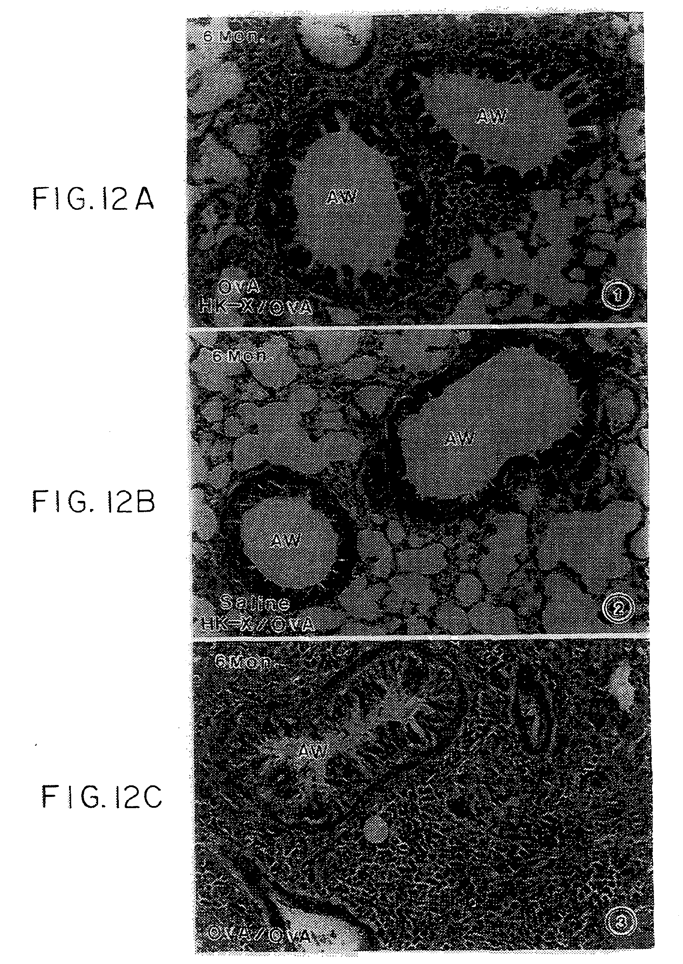

OVA immunized mice contained increased accumulations of collagen (blue color) around vessels and airways (see Fig. 12C). However, lungs treated with HK-X demonstrated a reduced level of collagen deposition (see Fig. 12A). In control mice that were administered HK-X in saline, the pulmonary tissue was free of inflammatory cells and fibrotic collagen deposits (see Fig. 12B).

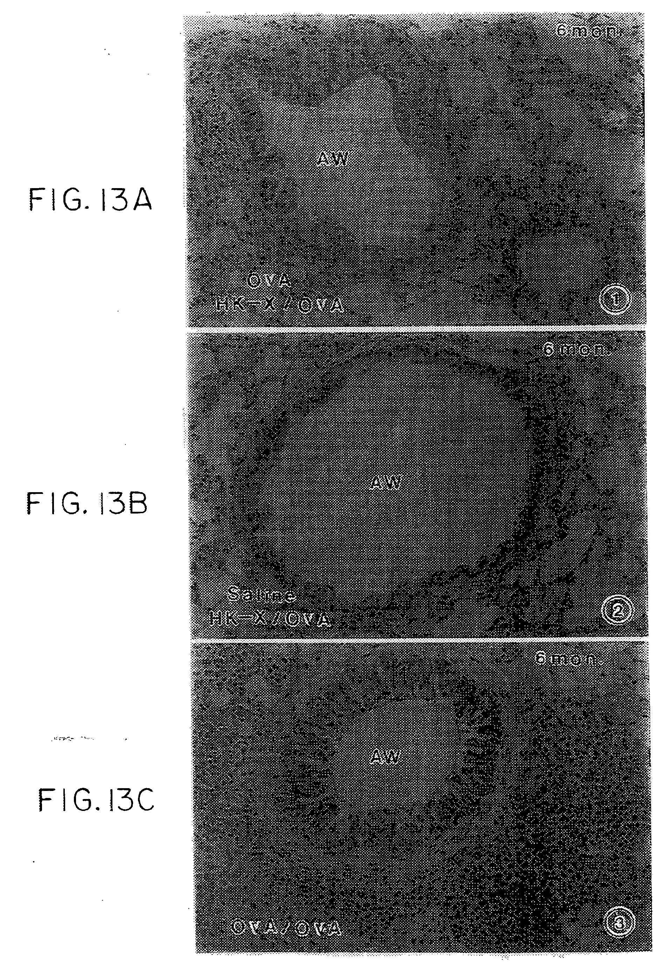

A similar pattern of results was observed when Alcian blue at pH 2.3 visualized mucus-containing cells. A very high proportion of epithelial cells in OVA immunized mice contained mucus granules (see Fig. 13C). By contrast, treatment with HK-X dramatically reduced the number of mucus containing cells in the airways (see Fig. 13A). In fact, the frequency was not different from that of control or unimmunized animals given on saline containing HK-X (see Fig. 13B).

### Effects of HK-X on Eosinophil and Neutrophil Recruitment into the Lungs:

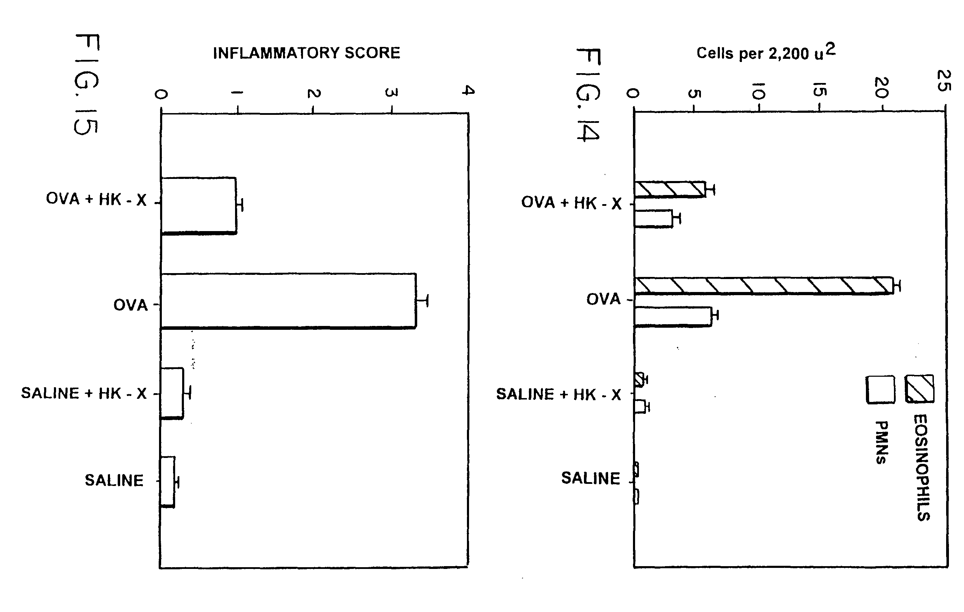

The effect of HK-X treatment on eosinophil and neutrophil recruitment into the lung is shown in Figure 14. Pre-treatment with HK-X reduced by 70% the number of infiltrating eosinophils and by 30% the number of infiltrating neutrophils into the lung tissue of OVA immunized and challenged mice, respectively. The mean number of eosinophils observed in saline treated lungs was 0.3 ± 0.1 cells per 2,200 µm². After HK-X treatment in saline only, the number of eosinophils in the lung tissues increased slightly, 0.75 ± 0.18 cells per 2,200 µm². In contrast, HK-X treatment significantly reduced the number of total inflammatory cellular infiltration in association with airways and blood vessels (Fig. 15).

### Mucus Accumulation in Lungs Reduced by HK-X Treatment:

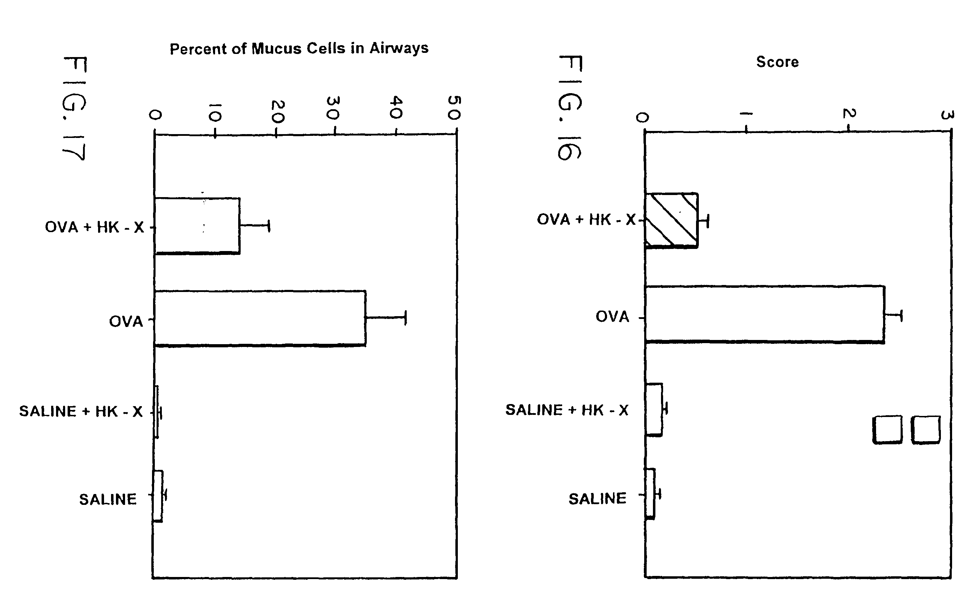

Following intranasal allergen challenges for 3 consecutive days, airway mucus plug scores and the number of airways mucus cells were determined. Airway mucus plug scores were assessed on a scale of 0-5, as described above in the *Methods*. Mucus cell numbers was semiquantitatively determined as described in Example 1. In the saline treated group, the mean airway mucus accumulation score was 0.1 ± 0.05 (see Fig. 16). In the OVA challenged group, the airway plug score was increased 23.3 fold to a score of 2.33 ± 0.17 (saline group vs. OVA group, p < 0.001; by Mann Whitney Rank Sum Test). Airway mucus plug scores or airway occlusion were reduced 78% by HK-X treatment, 15-30 min prior to OVA challenge (p < 0.001; by Mann Whitney Rank Sum Test). Similarly, the number of mucus cells in airways was reduced (see Fig. 17). Specifically, in OVA challenged lungs without HK-X treatment 34.66 ± 6.45% of the epithelial cells had differentiated into mucus secretion whereas, in HK-X treated lungs, only 11.24 ± 4.73% of cells contained mucus (p < 0.001; by Mann Whitney Rank Sum Test).

### The Level of Formation of Granuloma-like Structures in Chronic Asthmatic Lung:

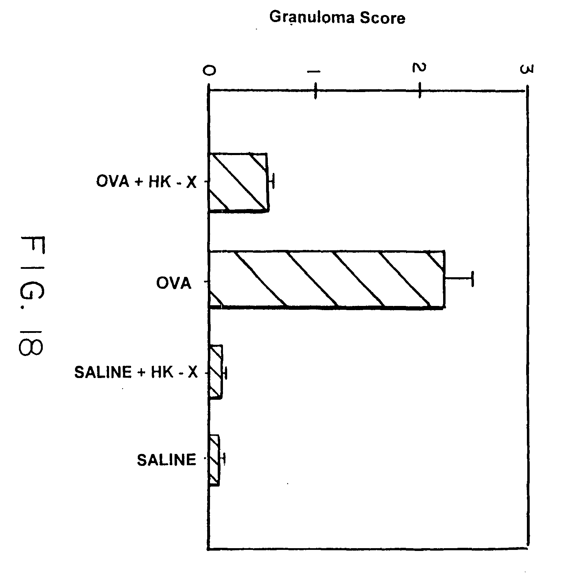

Granuloma formation was determined in lungs repeatedly challenged with both OVA and HK-X or OVA alone in animals immunized with OVA for 6 months. As shown in Fig. 18, there was a 3-fold reduction of granuloma formation in the lung by HK-X treatment (p < 0.001; by Mann Whitney Rank Sum Test).

These studies were designed to determine whether or not the administration of HK-X reduced airway inflammation and hyperactivity of mucus cells in a murine model of allergen-induced chronic asthma. In this model, mice were sensitized to OVA and subsequently exposed to OVA via intranasal route weekly for 5 months, and were further exposed to OVA intranasal challenge for three consecutive days. This allergen immunization and challenge regimen lead to a chronic airway infiltration of eosinophils and other types of inflammatory cells, accumulation of mucus in the airways and hyperplasia of mucus secreting cells.

Further, in this model the airway hypersecretion, hyperplasia of mucus cells, and recruitment of eosinophils and neutrophils are reduced by the administration of HK-X. These results indicate that HK-X is playing an important role in both the reduction of airway hypersecretion of mucus and the late-phase inflammation which occurs in this allergen-induced model of chronic asthma. Moderation of lung inflammation and reduction of mucus secretion and mucus cell differentiation by HK-X indicates that HK-X will be useful in the treatment of chronic asthma in humans.

The invention has been described in detail with reference to preferred embodiments thereof. However, it will be appreciated that, upon consideration of the present specification and drawings, those skilled in the art may make modifications and improvements.

## Claims

1. A use of a peptide having the formula f-Met-Leu-X where X is selected from the group consisting of
Tyr, Tyr-Phe, Phe-Phe and Phe-Tyr
for the preparation of a pharmaceutical for treating fibrosis in a mammal, wherein the fibrosis is due to pathological changes resulting from a condition selected from the group consisting of pulmonary fibrosis caused by chronic asthma, atherosclerosis, cirrhosis, glomerulosclerosis, chronic pancreatitis, coronary artery disease, trauma and surgical procedures.

2. The use of claim 1, wherein the fibrosis is due to pathological changes resulting from pulmonary fibrosis caused by chronic asthma.

3. The use of claim 1, wherein the fibrosis is due to pathological changes resulting from atherosclerosis.

4. The use of claim 1, wherein the fibrosis is due to pathological changes resulting from cirrhosis.

5. The use of claim 1, wherein the fibrosis is due to pathological changes resulting from glomerulosclerosis.

6. The use of claim 1, wherein the fibrosis is due to pathological changes resulting from chronic pancreatitis.

7. The use of claim 1, wherein the fibrosis is due to pathological changes resulting from coronary artery disease

8. The use of claim 1, wherein the fibrosis is due to pathological changes resulting from a condition selected from the group consisting of trauma and surgical procedures.

9. The use of claim 1, wherein the fibrosis is due to pathological changes resulting from trauma.

10. The use of claim 1, wherein the fibrosis is due to pathological changes resulting from surgical procedures.

11. The use of claim 9, wherein the fibrosis is due to pathological changes resulting from a condition selected from the group consisting of postoperative fibrosis perineurally in the dura or nerve roots following spinal surgery, tenolysis of injured or repaired tendons with adhesions, neurolysis of damaged or repaired peripheral nerves with adhesions, post-operative adhesions from gynecologic and abdominal surgeries, reparative surgery of the vas deferens or fallopian tubes for reversal of male or female sterilization, and surgical repair of other tubular structures such as urethra, intestine or esophagus.

## Patentansprüche

1. Verwendung eines Peptids, das die Formel f-Met-Leu-X aufweist, wobei X aus der aus
Tyr, Tyr-Phe, Phe-Phe und Phe-Tyr bestehenden Gruppe ausgewählt ist,
zur Herstellung eines Arzneimittels zur Behandlung von Fibrose bei einem Säuger, wobei die Fibrose auf pathologischen Veränderungen beruht, die sich aus einem Zustand ergeben, der aus der aus durch chronisches Asthma verursachten Lungenfibrose, Atherosklerose, Zirrhose, Glomerulosklerose, chronischer Pankreatitis, Koronararterienerkrankung, einem Trauma und chirurgischen Operationen bestehenden Gruppe ausgewählt ist.

2. Verwendung von Anspruch 1, wobei die Fibrose auf pathologischen Veränderungen beruht, die sich aus durch chronisches Asthma verursachter Fibrose ergeben.

3. Verwendung von Anspruch 1, wobei die Fibrose auf pathologischen Veränderungen beruht, die sich aus Atherosklerose ergeben.

4. Verwendung von Anspruch 1, wobei die Fibrose auf pathologischen Veränderungen beruht, die sich aus Zirrhose ergeben.

5. Verwendung von Anspruch 1, wobei die Fibrose auf pathologischen Veränderungen beruht, die sich aus Glomerulosklerose ergeben.

6. Verwendung von Anspruch 1, wobei die Fibrose auf pathologischen Veränderungen beruht, die sich aus chronischer Pankreatitis ergeben.

7. Verwendung von Anspruch 1, wobei die Fibrose auf pathologischen Veränderungen beruht, die sich aus einer Koronararterienerkrankung ergeben.

8. Verwendung von Anspruch 1, wobei die Fibrose auf pathologischen Veränderungen beruht, die sich aus einem Zustand ergeben, der aus der aus Trauma und chirurgischen Operationen bestehenden Gruppe ausgewählt ist.

9. Verwendung von Anspruch 1, wobei die Fibrose auf pathologischen Veränderungen beruht, die sich aus einem Trauma ergeben.

10. Verwendung von Anspruch 1, wobei die Fibrose auf pathologischen Veränderungen beruht, die sich aus chirurgischen Operationen ergeben.

11. Die Verwendung von Anspruch 9, wobei die Fibrose auf pathologischen Veränderungen beruht, die sich aus einem Zustand ergeben, der aus der aus postoperativer Fibrose perineural in der Dura oder den Nervenwurzeln als Folge einer Spinaloperation, Tenolyse von verletzten oder reparierten Sehnen mit Adhäsionen, Neurolyse von verletzten oder reparierten peripheren Nerven mit Adhäsionen, postoperativen Adhäsionen von gynäkologischen und abdominalen Operationen, wiederherstellenden Operationen vom Samenleiter oder Eileiter zur Umkehrung von männlicher oder weiblicher Sterilisation, und operativer Wiederherstellung anderer tubulärer Strukturen wie Urethra, Intestin oder Speiseröhre bestehenden Gruppe ausgewählt ist.

## Revendications

1. Utilisation d'un peptide de formule f-Met-Leu-X où X est choisi dans le groupe constitué par
Tyr, Tyr-Phe, Phe-Phe et Phe-Tyr
pour la préparation d'un agent pharmaceutique destiné au traitement d'une fibrose chez un mammifère, laquelle fibrose est due à des changements pathologiques résultant d'un état choisi dans le groupe constitué par la fibrose pulmonaire provoquée par un asthme chronique, l'athérosclérose, la cirrhose, la glomérulosclérose, la pancréatite chronique, une maladie coronarienne, un traumatisme, et des opérations chirurgicales.

2. Utilisation selon la revendication 1, dans laquelle la fibrose est due à des changements pathologiques résultant d'une fibrose pulmonaire provoquée par un asthme chronique.

3. Utilisation selon la revendication 1, dans laquelle la fibrose est due à des changements pathologiques résultant d'une athérosclérose.

4. Utilisation selon la revendication 1, dans laquelle la fibrose est due à des changements pathologiques résultant d'une cirrhose.

5. Utilisation selon la revendication 1, dans laquelle la fibrose est due à des changements pathologiques résultant d'une glomérulosclérose.

6. Utilisation selon la revendication 1, dans laquelle la fibrose est due à des changements pathologiques résultant d'une pancréatite chronique.

7. Utilisation selon la revendication 1, dans laquelle la fibrose est due à des changements pathologiques résultant d'une maladie coronarienne.

8. Utilisation selon la revendication 1, dans laquelle la fibrose est due à des changements pathologiques résultant d'un état choisi dans le groupe constitué par un traumatisme et des opérations chirurgicales.

9. Utilisation selon la revendication 1, dans laquelle la fibrose est due à des changements pathologiques résultant d'un traumatisme.

10. Utilisation selon la revendication 1, dans laquelle la fibrose est due à des changements pathologiques résultant d'opérations chirurgicales.

11. Utilisation selon la revendication 9, dans laquelle la fibrose est due à des changements pathologiques résultant d'un état choisi dans le groupe constitué par une fibrose postopératoire périneurale dans la dure-mère ou les racines nerveuses après opération spinale, une ténolyse de tendons blessés ou réparés présentant des adhérences, une neurolyse de nerfs périphériques endommagés ou réparés présentant des adhérences, des adhérences postopératoires suivant des opérations chirurgicales gynécologiques et abdominales, une chirurgie réparatrice du canal déférent ou des trompes de Fallope pour inverser une stérilisation mâle ou femelle, et une réparation chirurgicale d'autres structures tubulaires telles que l'urètre, l'intestin ou l'oesophage.
